# NEUE EUROPÄISCHE PATENTSCHRIFT

(11) **EP 2 999 752 B2**
(45) Veröffentlichungstag und Bekanntmachung des Hinweises auf die Entscheidung über den Einspruch: **31.07.2024**
(45) Hinweis auf die Patenterteilung: 29.03.2017
(21) Anmeldenummer: 14752556.2
(22) Anmeldetag: 29.07.2014
(51) Int. Cl.: C09C 1/64, C09C 1/00, A61K 8/25, A61K 8/26, A61Q 1/02, A61K 8/02, A61K 8/19, C09D 5/38, C09D 5/36, C09D 7/62

(54) **METALLISCHE GLANZPIGMENTE BASIEREND AUF SUBSTRATPLÄTTCHEN MIT EINER DICKE VON 1-50 NM**
METALLIC GLOSS PIGMENTS BASED ON FLAKY SUBSTRATES WITH A THICKNESS OF 1-50 NM
PIGMENTS BRILLANTS MÉTALLIQUES BASÉS SUR DES SUBSTRATS AYANT UNE ÉPAISSEUR DE 1-50 NM

(30) Priorität: 02.08.2013 EP 13003870
(43) Veröffentlichungstag der Anmeldung: 30.03.2016
(60) Teilanmeldung: 16000607.8
(73) Patentinhaber: Schlenk Metallic Pigments GmbH, 91154 Roth (DE)
(72) Erfinder: SHIMIZU, Kaiman, 91154 Roth (DE); PIECH, Fabian, 90530 Wendelstein (DE); HUBER, Adalbert, 64625 Bensheim (DE)
(74) Vertreter: Müller-Boré & Partner Patentanwälte PartG mbB
(86) Internationale Anmeldenummer: PCT/EP2014/002073
(87) Internationale Veröffentlichungsnummer: WO 2015/014484

(56) Entgegenhaltungen:
- WO-A1-2009/083176
- WO-A2-2005/049739
- DE-A1- 10 128 491
- DE-A1- 10 354 763
- DE-A1- 102007 062 942
- DE-A1- 19 836 810

## Beschreibung

Die vorliegende Erfindung betrifft metallische Glanzpigmente, ein Verfahren zu deren Herstellung sowie die Verwendung solcher metallischer Glanzpigmente.

Metallische Glanzpigmente beziehungsweise Metalleffektpigmente finden breite Anwendung in vielen Bereichen der Technik. Sie werden beispielsweise zur Einfärbung von Lacken, Druckfarben, Tinten, Kunststoffen, Gläsern, keramischen Produkten und Zubereitungen der dekorativen Kosmetik eingesetzt. Von besonderer wirtschaftlicher Bedeutung ist die Verwendung von metallischen Glanzpigmenten in Automobillackierungen. Aufgrund ihrer nicht kopierbaren optischen Effekte werden sie auch in der Herstellung fälschungssicherer Wertschriften und Dokumente wie Geldscheine, Schecks, Bank- und Kreditkarten, Eintrittskarten und Tickets, eingesetzt. Sie zeichnen sich vor allem durch ihren reizvollen winkelabhängigen Farbeindruck (Goniochromie) und ihren metallartig wirkenden Glanz aus.

Bei gewöhnlichen Pigmenten entsteht ein Farbeindruck lediglich durch Absorption bestimmter Wellenlängen einfallenden Lichts und Streureflexion. Gängige metallische Effektpigmente reflektieren das einfallende Licht in hohem Maße und erzeugen einen Hell-Dunkel-Flop, aber keinen Farbeindruck. Bei speziellen metallischen Glanzpigmenten entsteht jedoch aufgrund optischer Interferenzeffekte ein Farbeindruck. Derartige metallische Glanzpigmente, die in der Regel auf mindestens einfach beschichteten plättchenförmigen Substraten beruhen, zeigen Interferenzeffekte durch Überlagerung von verschieden gebrochenen und reflektierten Lichtstrahlen. Auf die ebene Oberfläche der beschichteten Substrate einfallendes weißes Licht wird zum Teil an der äußeren Oberfläche der Beschichtung reflektiert. Der andere Teil wird gebrochen und an Grenzflächen, beispielsweise zwischen Beschichtung und Substratoberfläche, reflektiert und erneut gebrochen. Daher kommt es zur Überlagerung von Lichtstrahlen unterschiedlicher Phase. Durch Interferenz des reflektierten Lichts entsteht ein Farbeindruck. Aufgrund der Abhängigkeit der Phasendifferenz vom Einfalls-/Beobachtungswinkel ist auch der Farbeindruck winkelabhängig. Dieser Effekt des Farbwechsels zwischen verschiedenen Reflexionswinkeln wird als Farbflop bezeichnet. Die Phasendifferenz wird u.a. durch die Dicke der Beschichtung(en) beeinflusst, wodurch der entstehende Farbeindruck über die Beschichtungsdicke eingestellt werden kann.

EP-A-0 033 457 beschreibt Pigmente auf der Basis von Eisenoxid-beschichteten Aluminiumplättchen, die im Glanzwinkel (Reflexionswinkel mit höchster Helligkeit) goldene bis rote Farbtöne aufweisen.

DE 94 00 447 U1 beschreibt Glanzpigmente auf der Basis von nitrierten Metall(oxid)plättchen, die eine hohe Härte aufweisen und für die Verwendung in Farben und Lacken, in der Kosmetikbranche und für das Färben von Kunststoffen geeignet sind.

WO 2004/113455 beschreibt ein Verfahren zur Herstellung eines Pigments, umfassend ein SiO_{z} Kernmaterial und zumindest eine dielektrische Schicht durch Mikrowellenabscheidung eines Metalloxids aus einer wässrigen Lösung.

WO 2005/049739 offenbart Effektpigmente mit einem Aluminium- oder Aluminiumlegierungskern und einer den Aluminium-oder Aluminiumlegierungskern umhüllenden Aluminiumoxid- oder Aluminiumoxid/hydroxid-haltigen Schicht, erhältlich durch nasschemische Oxidation plättchenförmiger Aluminium- oder Aluminiumlegierungs-Pigmente, wobei der Gehalt an metallischem Aluminium im Aluminium- oder Aluminiumlegierungskern nicht mehr als 90 Gew.-%, bezogen auf das Gesamtgewicht des Pigments, beträgt.

DE 198 36 810 A1 offenbart Mehrschichtpigmente auf Basis von plättchenförmigen Metallpigmenten, hergestellt indem die Metallpigmente ausschliesslich nasschemisch im Eintopfverfahren belegt werden, wobei die Metallpigmente zunächst in Wasser suspendiert, bei einem pH-Wert von 6-11 mit einer amorphen glasartigen Schicht und nachfolgend bei einem pH-Wert von <4 mit ein oder mehreren Metalloxiden bzw. Metalloxidgemischen belegt werden.

Die aus dem Stand der Technik bekannten Pigmente weisen jedoch erhebliche Mängel auf. So ist das Deckvermögen bekannter Pigmente zwar für gewisse Anwendungen ausreichend. Es wäre allerdings aus Effizienzgründen wünschenswert, metallische Glanzpigmente mit höherem Deckvermögen bereitzustellen. Insbesondere bei Automobillacken besteht die Forderung nach immer dünneren Lackschichten, welche durch Pigmente mit höherem Deckvermögen erzielt werden können. Darüber hinaus weisen metallische Glanzpigmente auf der Basis Metalloxid-beschichteter Aluminiumplättchen mitunter nachteilige Sicherheitseigenschaften auf. So können derartige Pigmente entflammbar und sogar explosionsgefährlich sein. Insbesondere mit Eisenoxid reagiert Aluminium besonders heftig (Thermitreaktion). Diese Eigenschaften bekannter metallischer Glanzpigmente auf Aluminiumbasis schränken die Arbeitssicherheit ein.

Somit liegt der vorliegenden Erfindung die Aufgabe zugrunde, ein kostengünstiges metallisches Glanzpigment, welches ein hohes Deckvermögen und eine geringes anwendungstechnisches Brennverhalten aufweisen soll, bereitzustellen.

Diese Aufgabe wird durch die in den Ansprüchen gekennzeichneten Ausführungsformen gelöst.

Insbesondere werden gemäß Anspruch 1 metallische Glanzpigmente auf der Basis von beschichteten Aluminium-Substratplättchen bereitgestellt,
wobei die Aluminium-Substratplättchen eine Dicke von 5 bis 30 nm aufweisen, monolithisch aufgebaut und gegebenenfalls passiviert sind und von einer Beschichtung B umhüllt sind,
wobei die Beschichtung B aus Fe₂O₃ in einer Dicke von 50 bis 300 nm aufgebaut ist, und
wobei zwischen der Oberfläche der Aluminium-Substratplättchen und der Beschichtung B eine weitere, die Substratplättchen umhüllende Beschichtung A aus SiO₂ in einer Dicke von 5 bis 50 nm vorhanden ist,
wobei die Substratplättchen eine weitere Beschichtung C, die von der darunterliegenden Beschichtung B verschieden ist, aus mindestens einem Metalloxid(hydrat), ausgewählt aus Silicium(di)oxid, Siliciumoxidhydrat, Aluminiumoxid, Aluminiumoxidhydrat, Zinkoxid, Zinnoxid, Titandioxid, Zirkonoxid oder Chrom(III)oxid, aufweisen, und
wobei als Aluminium-Substratplättchen Al-VMPs verwendet werden.

Im Rahmen der vorliegenden Erfindung werden der Einfachheit halber Si, B und Ge den Metallen zugeordnet.

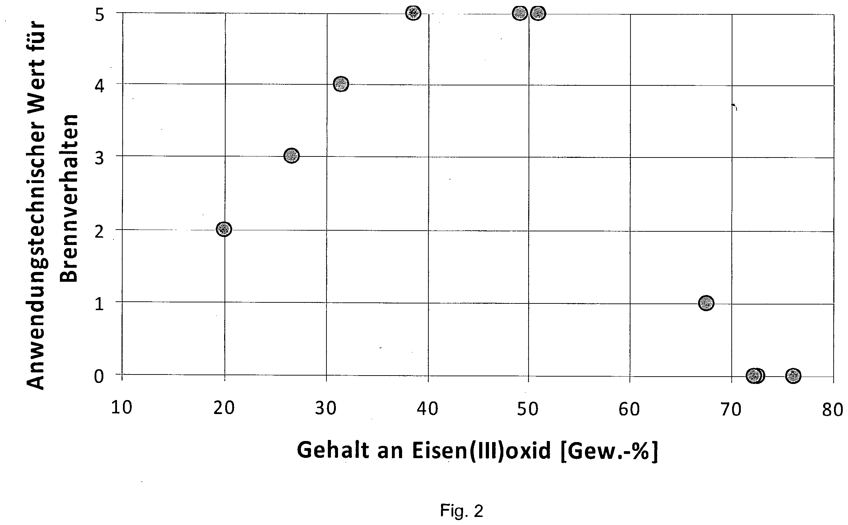

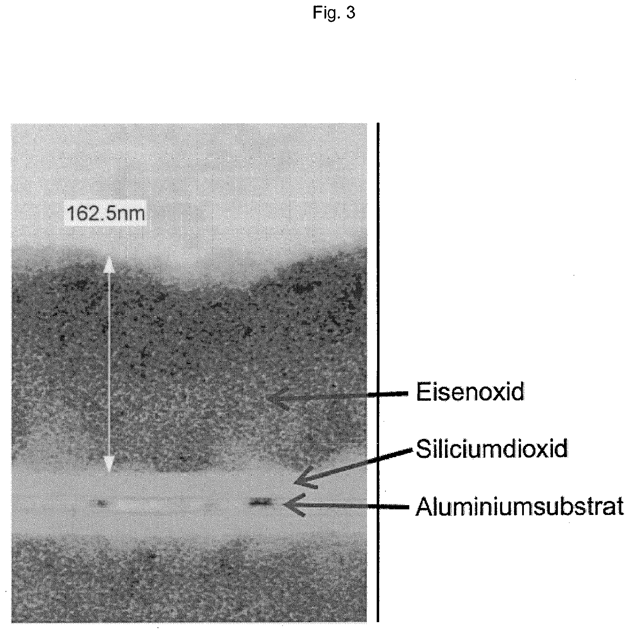

Die Aluminium-Substratplättchen weisen eine durchschnittliche Dicke von höchstens 50 nm, vorzugsweise weniger als 30 nm, besonders bevorzugt höchstens 25 nm, beispielsweise höchstens 20 nm auf. Die durchschnittliche Dicke der Aluminium-Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke der Aluminium-Substratplättchen sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf. Herstellungsbedingt können allerdings Schwankungen der Dicke innerhalb eines Plättchens auftreten. Diese sollten vorzugsweise nicht mehr als ±25%, bezogen auf die durchschnittliche Dicke des betrachteten Plättchens, besonders bevorzugt höchstens ±10%, insbesondere bevorzugt höchstens ±5% betragen. Unter der durchschnittlichen Dicke ist hier das Zahlenmittel aus maximaler und minimaler Dicke zu verstehen. Die Ermittlung der minimalen und maximalen Schichtdicke erfolgt durch Ausmessen auf Grundlage einer transmissionselektronenmikroskopischen (TEM) Aufnahme eines (beschichteten) Substratplättchens (vgl. Figuren 2 und 3). Nachdem die Farbe der beschichteten Substratplättchen linear von der Schichtdicke abhängig ist, wird durch eine präzise und einheitlich eingestellte Dicke der unbeschichteten Aluminium-Substratplättchen eine einheitliche Farbwirkung sichergestellt.

Unter Plättchen bzw. Flakes werden solche im Rahmen der vorliegenden Erfindung verstanden, die ein Dicken-/Längenverhältnis von mindestens 10:1, vorzugsweise darüber, aufweisen.

Bezüglich der Schwankung der Schichtdicke und der Bestimmung der (durchschnittlichen) Schichtdicke gilt das Vorstehende analog auch für die Dicken der Beschichtungen A sowie B und C.

Soweit hier auf die "Dicke" einer Beschichtung oder eines Aluminium-Substratplättchens Bezug genommen wird, gilt damit die durchschnittliche Dicke als bezeichnet, sofern an betreffender Stelle nichts anderes bestimmt ist.

Die Aluminium-Substratplättchen sind monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb der Substratplättchen Gefügewechsel auftreten können (siehe Figur 2). Die Aluminium-Substratplättchen sind vorzugsweise homogen aufgebaut, d.h. dass innerhalb der Plättchen kein Konzentrationsgradient auftritt. Insbesondere sind die Aluminium-Substratplättchen nicht schichtartig aufgebaut und weisen keine darin verteilten Teilchen oder Partikel auf. Sie weisen insbesondere keinen Kern-Schale Aufbau auf, wobei die Schale beispielsweise aus einem für die Substratplättchen geeigneten Material und der Kern aus einem anderen Material, beispielsweise einem Siliciumoxid, besteht. Durch ihren einfachen Aufbau sind die Substratplättchen kostenkünstig und effizient herzustellen. Im Gegensatz dazu bedingt ein komplexerer, nicht-monolithischer Aufbau der Substratplättchen einen erschwerten, zeit- und kostenintensiven Herstellungsprozess.

Der Massenanteil des Aluminium-Substratplättchens an dem beschichteten Substratplättchen beträgt vorzugsweise höchstens 20 Gew.-%, besonders bevorzugt höchstens 15 Gew.-%, beispielsweise höchstens 10 Gew.-%. Allerdings sollte der Massenanteil der Aluminium-Substratplättchen nicht unterhalb von 0,1 Gew.-%, vorzugsweise nicht unter 0,5 Gew.-% oder 1 Gew.-% fallen.

Durch die geringe Dicke bzw. den geringen Massenanteil der Aluminium-Substratplättchen weist das erfindungsgemäße metallische Glanzpigment ein besonders hohes Deckvermögen auf.

Das erfindungsgemäße Glanzpigment weist vorzugsweise einen Gesamtfarbabstand ΔE von höchstens 10, besonders bevorzugt höchstens 5, insbesondere höchstens 3 auf. Die Messung von ΔE erfolgt hierbei nach DIN 55987, wobei auf jeweils eine schwarze und eine weiße Oberfläche eine Lackschicht, die das erfindungsgemäße metallische Glanzpigment mit einem Massenanteil von 18 Gew.-% (Trockengewicht) enthält, aufgetragen wird. Die Schichtdicke der getrockneten Lackierung beträgt 15 µm. Danach wird der Gesamtfarbabstand ΔE zwischen den Lackierungen auf weißem und schwarzem Grund bestimmt.

Insofern ist die vorliegende Erfindung in einer weiteren unabhängigen Ausführungsform auch auf Glanzpigmente gerichtet, die einen Gesamtfarbabstand ΔE von höchstens 10, besonders bevorzugt höchstens 5, insbesondere höchstens 3 aufweisen.

Abgesehen von der Dicke ist die Größe der unbeschichteten Aluminium-Substratplättchen nicht kritisch und kann auf den jeweiligen Anwendungszweck abgestimmt werden. In der Regel haben die Plättchen mittlere größte Durchmesser von etwa 2 bis 200 µm, insbesondere etwa 5 bis 100 µm. Der d50-Wert der unbeschichteten Aluminium-Substratplättchen beträgt für die Verwendung in Automobillacken vorzugsweise 5 bis 50 µm, besonders bevorzugt 10 bis 30 µm, kann für andere Verwendungen, beispielsweise als Industrielack, aber auch bei Werten von etwa 70 µm liegen.

Hierin wird der d50-Wert, soweit nicht anders angegeben, mit einem Gerät des Typs Sympatec Helos mit Quixel-Nassdispergierung bestimmt. Dabei wird zur Probenvorbereitung die zu untersuchende Probe für eine Dauer von 3 Minuten in Isopropanol vordispergiert.

Die beschichteten Aluminium-Substratplättchen weisen vorzugsweise eine Dicke von 70 bis 500 nm, besonders bevorzugt 100 bis 400 nm, insbesondere bevorzugt 150 bis 320 nm, beispielsweise 180 bis 290 nm, auf. Aufgrund der geringen Dicke der Substratplättchen weist das erfindungsgemäße metallische Glanzpigment ein besonders hohes Deckvermögen auf. Die geringe Dicke der beschichteten Aluminium-Substratplättchen wird insbesondere dadurch erzielt, dass die Dicke der unbeschichteten Substratplättchen gering ist, aber auch dadurch, dass die Dicken der Beschichtungen A und C auf einen möglichst kleinen Wert eingestellt werden. Nachdem die Dicke von Beschichtung B den Farbeindruck des metallischen Glanzpigments bestimmt, ist bei einem festgelegten gewünschten Farbeffekt diesbezüglich kein Spielraum gegeben.

Bisher wurde davon ausgegangen, dass ausschließlich nicht lichtdurchlässige (opake) Materialien als Substratplättchen geeignet sind. Zudem wurde angenommen, dass unbeschichtete Substratteilchen eine gewisse Dicke nicht unterschreiten dürfen, um deren (teilweise) Lichtdurchlässigkeit zu vermeiden, was gemäß dieser Annahme zu einem stark herabgesetztem Deckvermögen des resultierenden Glanzpigments führen würde.

Überraschenderweise wurde jedoch festgestellt, dass mit (teilweise oder ganz lichtdurchlässigen) Aluminium-Substratplättchen mit einer Schichtdicke von höchstens 50 nm, vorzugsweise höchstens oder kleiner 30 nm, metallische Glanzpigmente hergestellt werden können, die ein deutlich höheres Deckvermögen aufweisen als gewöhnliche metallische Glanzpigmente. Wahrscheinlich liegt der Grund dafür darin, dass durch die geringe Dicke der beschichteten Aluminium-Substratplättchen eine höhere Flächenabdeckung des metallischen Glanzpigments erzielt wird. Nachdem die beschichteten Substratplättchen dünn sind, kann mit der gleichen Masse Pigment eine höhere Fläche abgedeckt werden. Durch diesen vorteilhaften Effekt wird die höhere Lichtdurchlässigkeit dünner, ganz oder teilweise lichtdurchlässiger Substratplättchen überkompensiert, so dass letztendlich gegenüber metallischen Glanzpigmenten mit dicken Substratplättchen ein höheres Deckvermögen erzielt wird.

Aluminiumplättchen können unter anderem durch Herausstanzen aus Aluminiumfolie oder nach gängigen Mahl- und Verdüsungstechniken hergestellt werden. So sind beispielsweise Aluminiumplättchen aus dem Hallverfahren, einem Nassmahlverfahren, erhältlich.

Als Aluminium-Substratplättchen werden erfindungsgemäß sog. *vacuum metallized pigments* (VMP) verwendet.

Die Aluminiumplättchen können passiviert sein, beispielsweise durch Eloxieren (Oxidschicht) oder Chromatieren.

VMPs können durch das Freisetzen von Aluminium von metallisierten Folien gewonnen werden. Sie zeichnen sich durch eine besonders geringe Dicke der Substratplättchen im Bereich von 5 bis 50 nm, vorzugsweise bis oder kleiner 30 nm, und durch eine besonders glatte Oberfläche mit erhöhter Reflektivität aus.

Das erfindungsgemäße metallische Glanzpigment kann sowohl ein Glanzpigment des leafing- als auch des non-leafing-Typs sein. Bevorzugt ist das metallische Glanzpigment ein Glanzpigment des non-leafing-Typs.

Erfindungsgemäß sind die beschichteten Aluminium-Substratplättchen von einer Beschichtung B umhüllt. Zwischen der Beschichtung B und der Substratoberfläche weisen die beschichteten Substratplättchen eine Beschichtung A auf. Zudem weisen die Substratplättchen eine weitere Beschichtung C, die von der darunterliegenden Schicht B verschieden ist, auf.

Allgemein ist eine Beschichtung eines Teils der Oberfläche der beschichteten Substratplättchen ausreichend, um ein Glanzpigment zu erhalten. So können beispielsweise lediglich die obere und/oder untere Seite der Plättchen beschichtet sein, wobei die Seitenfläche(n) ausgespart sind. Erfindungsgemäß ist allerdings die gesamte Oberfläche der gegebenenfalls passivierten Substratplättchen, einschließlich der Seitenflächen, von Beschichtung B bedeckt. Die Substratplättchen sind also vollständig von Beschichtung B umhüllt. Dies verbessert die optischen Eigenschaften des erfindungsgemäßen Pigments und erhöht die mechanische und chemische Belastbarkeit der beschichteten Substratplättchen. Das Vorstehende gilt auch für die Schicht A und vorzugsweise auch für die Schicht C.

Obwohl jeweils mehrere Beschichtungen A, B und C vorhanden sein können, weisen die beschichteten Substratplättchen vorzugsweise jeweils nur eine Beschichtung A, B und C auf.

Die Beschichtung B ist aus Fe₂O₃, einem hochbrechenden Metalloxid, aufgebaut.

Die Beschichtung B weist eine Dicke von 50 bis 300 nm auf.

Das Verhältnis der Dicke von Beschichtung B zur Dicke der unbeschichteten Aluminium-Substratplättchen beträgt vorzugsweise mindestens mindestens 2, beispielsweise 4, 8 oder 10. Grundsätzlich muss für dieses Verhältnis keine Obergrenze eingehalten werden, es sollte aber aus praktischen Gründen höchstens 1000, vorzugsweise höchstens 500 betragen. Die durchschnittliche Dicke einer Beschichtung bzw. eines Substratplättchens bestimmt sich aus dem arithmetischen Mittel der maximalen und minimalen Dicke der Beschichtung/des Substratplättchens.

Soweit hier auf "Substratplättchen", ohne Unterscheidung, ob diese beschichtet sind oder nicht, Bezug genommen wird, gelten damit unbeschichtete Substratplättchen als bezeichnet, sofern an betreffender Stelle nichts anderes bestimmt ist.

Erfindungsgemäß ist zwischen der Oberfläche der Aluminium-Substratplättchen und Beschichtung B eine weitere Beschichtung A aus SiO₂, einem niedrigbrechenden Metalloxid, in einer Dicke von 5 bis 50 nm vorhanden.

Gelegentlich weisen die Metalloxide, welche für die Beschichtungen A, B und C eingesetzt werden können, einen gewissen Anteil an Nebenbestandteilen und/oder Verunreinigungen auf. Zu typischen Nebenbestandteilen von Metalloxiden zählen insbesondere Metallhydroxide. So kann beispielsweise eine Beschichtung aus Eisenoxid einen gewissen Anteil an Eisenhydroxid enthalten.

Hier bezeichnen die Begriffe "hochbrechend" und "niedrigbrechend" jeweils Materialien mit einem hohen bzw. niedrigen Brechungsindex. Hochbrechende Materialien weisen einen Brechungsindex von mindestens 1,9, bevorzugt mindestens 2,0 und besonders bevorzugt mindestens 2,4 auf. Niedrigbrechende Materialien weisen einen Brechungsindex von höchstens 1,8, bevorzugt höchstens 1,6 auf.

Hier bedeutet der Begriff "im Wesentlichen", sofern er auf einen Bestandteil einer Zusammensetzung angewandt wird, dass die Zusammensetzung zu mindestens 95 Gew.-%, bevorzugt zu mindestens 99 Gew.-%, insbesondere bevorzugt zu mindestens 99 Gew.-%, beispielsweise zu etwa 100 Gew.-% aus dem bezeichneten Bestandteil aufgebaut ist.

Für Beschichtung B vorzusehende hochbrechende Metalloxide sind selektiv lichtabsorbierende (d.h. farbige) Metalloxide aus Eisen(III)oxid (α- und γ-Fe₂O₃ rot).

Beschichtung B kann einen selektiv absorbierenden Farbstoff enthalten, vorzugsweise 0,001 bis 5 Gew.-%, besonders bevorzugt 0,01 bis 1 Gew.-%. Geeignet sind organische und anorganische Farbstoffe, die sich stabil in eine Metalloxidbeschichtung einbauen lassen.

Die Beschichtung A aus SiO₂ weist eine Dicke von 5 bis 50 nm, bevorzugt 5 bis 20 nm, auf. Vorzugsweise beträgt der Abstand zwischen der Oberfläche der Substratplättchen und der inneren Oberfläche von Beschichtung B höchstens 100 nm, besonders bevorzugt höchstens 50 nm, insbesondere bevorzugt höchstens 20 nm. Dadurch, dass die Dicke von Beschichtung A/der Abstand zwischen der Oberfläche der Substratplättchen und Beschichtung B im oben angegebenen Bereich liegt, kann sichergestellt werden, dass die beschichteten Substratplättchen des erfindungsgemäßen metallischen Glanzpigments ein hohes Deckvermögen und damit einen möglichst kleinen ΔE-Wert aufweisen.

Gemäß der vorliegenden Erfindung weisen die Substratplättchen eine weitere Beschichtung C aus einem Metalloxid(hydrat), die von der darunterliegenden Beschichtung B verschieden ist, ausgewählt aus Silicium(di)oxid, Siliciumoxidhydrat, Aluminiumoxid, Aluminiumoxidhydrat, Zinkoxid, Zinnoxid, Titandioxid, Zirkonoxid und Chrom(III)oxid, auf. Bevorzugt ist Siliciumdioxid.

Die Beschichtung C weist vorzugsweise eine Dicke von 10 bis 500 nm, besonders bevorzugt 50 bis 300 nm auf. Durch das Vorsehen der Beschichtung C, beispielsweise auf Basis von TiO₂, kann eine bessere Interferenz erzielt werden, wobei ein hohes Deckvermögen gewährleistet bleibt.

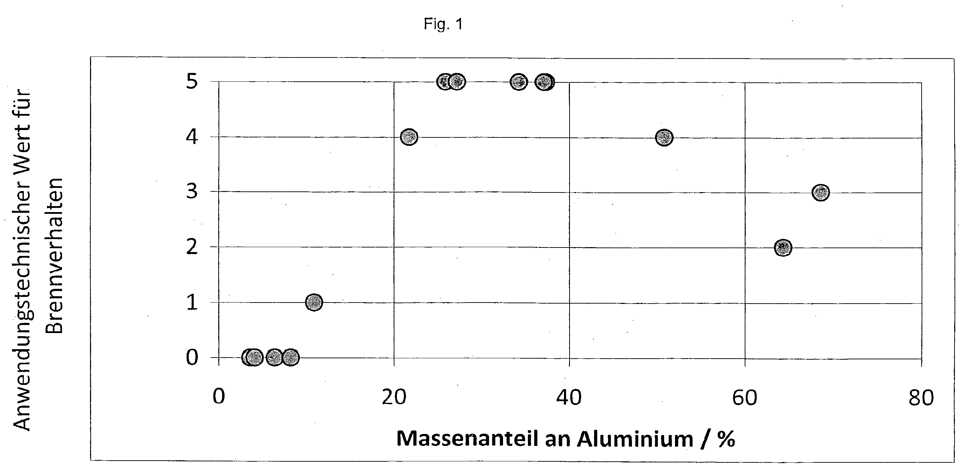

In dem erfindungsgemäßen metallischen Glanzpigment und den beschichteten Substratplättchen beträgt das Stoffmengenverhältnis α von Sauerstoff, der nicht an Aluminium gebunden ist, zu Aluminium vorzugsweise mindestens 3, besonders bevorzugt mindestens 4, insbesondere bevorzugt mindestens 5. Wenn α mindestens 3 beträgt, wird vermieden, dass in den beschichteten Substratplättchen ein Stoffmengenverhältnis von Sauerstoff, der nicht an Aluminium gebunden ist, zu Aluminium im stöchiometrischen Verhältnis von 3/2 (mol/mol) vorliegt. Ein Gemisch von Aluminium und Sauerstoffverbindungen, insbesondere Fe₂O₃, in dem das Stoffmengenverhältnis α im Bereich von 3/2 liegt, kann aufgrund der hohen Oxophilie von Aluminiummetall stark exotherm reagieren, unter Umständen mit Explosionwirkung (Aluminothermie, Thermitreaktion). Daher kann ein Gemisch mit α im Bereich von 3/2 eine Sicherheitsgefährdung darstellen. Die Reaktionsfähigkeit eines derartigen Gemisches kann jedoch dadurch herabgesetzt werden, dass das Verhältnis α auf einen Wert, der entweder deutlich größer oder deutlich kleiner als 3/2 ist, eingestellt wird (siehe Figur 1).

Um einen kleinen Wert für α zu erzielen, müsste der Aluminiumgehalt hoch eingestellt werden. Dies wäre mit dem Nachteil verbunden, dass die Dicke der Substratplättchen aus Aluminium groß eingestellt werden müsste, was dazu führen würde, dass das Deckvermögen eines derartigen Glanzpigments stark herabgesetzt wäre.

Somit wird das Stoffmengenverhältnis α vorzugsweise auf einen Wert oberhalb von 3/2, nämlich ≥ 3, eingestellt. Dadurch zeigen die beschichteten Substratplättchen und das erfindungsgemäße metallische Glanzpigment keine oder zumindest nur eine sehr geringe Reaktivität bei gleichzeitig hohem Deckvermögen.

Die Reaktivität des Glanzpigments ist auch dann unterdrückt oder vernachlässigbar gering, wenn der Massenanteil von Eisen(III)oxid, wenn als Beschichtung B vorgesehen, in Kombination mit einer Schicht C, ausgewählt aus TiO₂, SnO₂ oder Al₂O₃, in den beschichteten Substratplättchen hoch ist, vorzugsweise mindestens 65 Gew.-%, besonders bevorzugt mindestens 70 Gew.-%, insbesondere bevorzugt mindestens 75 Gew.-%. Der Gehalt an Eisen(III)oxid sollte jedoch nicht mehr als 99 Gew.-% betragen, vorzugsweise nicht mehr als 97 Gew.-%.

Des Weiteren ist die Reaktivität des Glanzpigments auch dann unterdrückt oder vernachlässigbar gering, wenn in dem metallischen Glanzpigment der Gehalt an Sauerstoff, der nicht an Aluminium gebunden ist, mindestens 50 mol-%, vorzugsweise mindestens 52,5 mol-%, besonders bevorzugt mindestens 55 mol-%, beispielsweise mindestens 57 mol-% beträgt. Der Stoffmengenanteil an Sauerstoff, der nicht an Aluminium gebunden ist, sollte jedoch nicht mehr als 59 mol-% betragen.

Das erfindungsgemäße Verfahren zur Herstellung des metallischen Glanzpigments umfasst die Schritte des Bereitstellens von gegebenenfalls passivierten Aluminium-Substratplättchen, des Beschichtens der Aluminium-Substratplättchen durch hydrolytische Zersetzung einer oder mehrerer organischer Metallverbindungen und/oder durch Fällung eines oder mehrerer gelöster Metallsalze.

Zur Erzeugung von Beschichtung A werden zweckmäßigerweise organische Metallverbindungen (vorzugsweise organische Siliciumverbindungen), bei denen die organischen Reste über Sauerstoffatome an die Metalle gebunden sind, in Gegenwart der Substratplättchen und eines organischen Lösemittels, in welchem die Metallverbindungen löslich sind, hydrolysiert. Hierfür sind eine Vielzahl von organischen Lösemitteln geeignet, bevorzugt ist Isopropanol. Im Falle von SiO₂ kann das Erzeugen der Beschichtung A auch im wässrigen Medium erfolgen.

Bevorzugte Beispiele für die organischen Metallverbindungen sind die Acetylacetonate und insbesondere Alkoholate, vor allem C₁-C₄-Alkanolate, z.B. Tetraethoxysilan (Tetraethylorthosilikat, TEOS).

Die Hydrolyse wird vorzugsweise in Gegenwart einer Base oder einer Säure als Katalysator durchgeführt. Hierfür eignen sich z. B. neben Alkalilaugen wie Natronlauge insbesondere wässrige Ammoniaklösungen. Geeignete saure Katalysatoren sind beispielsweise Phosphorsäure und organische Säuren wie Essigsäure und Oxalsäure.

Wasser muss mindestens in der stöchiometrisch für die Hydrolyse erforderlichen Menge vorliegen, bevorzugt ist jedoch die 2 bis 100-fache, insbesondere die 5 bis 20-fache Menge.

Bezogen auf die eingesetzte Wassermenge gibt man in der Regel 3 bis 40 Vol.-%, vorzugsweise 5 bis 30 Vol.-%, einer 25 Gew.-%igen wässrigen Ammoniaklösung zu.

Für die Temperaturführung hat es sich als vorteilhaft erwiesen, das Reaktionsgemisch innerhalb von 10 bis 48 h schrittweise auf Rückflusstemperatur zu erhitzen. Bei Verwendung von iso-Propanol als Lösungsmittel wird das Gemisch zum Beispiel bevorzugt zunächst 4 bis 20 h bei 40 °C, dann 4 bis 20 h bei 60 °C und zum Schluss 2 bis 8 h bei 80 °C gerührt.

Verfahrenstechnisch erfolgt das Beschichten von Substratplättchen mit einer Beschichtung A zweckmäßigerweise wie folgt:
Aluminium-Substratplättchen, organisches Lösungsmittel, Wasser und Katalysator (Säure oder bevorzugt Base, insbesondere z.B. eine wässrige Ammoniaklösung) werden vorgelegt, wonach die zu hydrolysierende Metallverbindung, als Reinstoff oder gelöst, z.B. als 30 bis 70, bevorzugt 40 bis 60 Vol.-%ige Lösung im organischen Lösemittel, zugegeben wird. Erfolgt die Zugabe der Metallverbindung in einem Schritt, dann wird die Suspension anschließend wie vorstehend beschrieben unter Rühren erhitzt. Die Metallverbindung kann aber auch bei erhöhter Temperatur kontinuierlich zudosiert werden, wobei Wasser und Ammoniak vorgelegt oder ebenfalls kontinuierlich zudosiert werden können. Nach beendeter Beschichtung wird das Reaktionsgemisch wieder auf Raumtemperatur abgekühlt.

Um eine Agglomeratbildung während des Beschichtungsvorgangs zu verhindern, kann die Suspension einer starken mechanischen Beanspruchung wie Pumpen, kräftigem Rühren oder Einwirken von Ultraschall unterzogen werden.

Gegebenenfalls kann der Beschichtungsschritt ein- oder mehrfach wiederholt werden. Sollte die Mutterlauge milchig trüb aussehen, so empfiehlt es sich, diese vor einer weiteren Beschichtung auszutauschen.

Die Isolierung der mit Beschichtung A ummantelten Aluminium-Substratplättchen kann in einfacher Weise durch Abfiltrieren, Waschen mit organischem Lösungsmittel, vorzugsweise den auch als Lösemittel verwendeten Alkoholen, und anschließendes Trocknen (üblicherweise 2 bis 24 h bei 20 bis 200 °C) erfolgen.

Zum Aufbringen der Metalloxidschichten (B) können α-Eisenoxidschichten durch hydrolytische Zersetzung von Eisen(III)salzen wie Eisen (III)-chlorid und -sulfat und anschließendes Überführen der gebildeten hydroxidhaltigen Schichten durch Tempern in die Oxidschichten aufgebracht werden. Das Tempern erfolgt vorzugsweise bei einer Temperatur von 250 bis 550°C für eine Dauer von 5 bis 60 Minuten, vorzugsweise 350 bis 450°C für eine Dauer von 10 bis 30 Minuten.

Die Beschichtung C kann wie für die Beschichtungen A und B beschrieben aufgebracht werden.

Mit Hilfe des erfindungsgemäßen Herstellungsverfahrens können die beschichteten Substratplättchen in einfacher Weise in großen Mengen reproduzierbar hergestellt werden. Es werden vollständig umhüllte Pigmentteilchen mit hoher Qualität der einzelnen Beschichtungen (homogen, filmartig) erhalten.

Darüber hinaus betrifft die vorliegende Erfindung in einem weiteren Aspekt die Verwendung der vorstehend beschriebenen metallischen Glanzpigmente zur Einfärbung von Lacken, Druckfarben, Tinten, Kunststoffen, Gläsern, keramischen Produkten und Zubereitungen der dekorativen Kosmetik.

Die erfindungsgemäßen Glanzpigmente eignen sich vorteilhaft für viele Zwecke, wie zur Einfärbung von Kunststoffen, Gläsern, keramischen Produkten, Zubereitungen der dekorativen Kosmetik und insbesondere von Tinten, Druckfarben und Sicherheitsdruckfarben und vor allem von Lacken, beispielsweise für die Automobilindustrie.

Für diese Anwendungszwecke lassen sich die erfindungsgemäßen Pigmente auch vorteilhaft in Abmischung mit transparenten und deckenden Weiß-, Bunt- und Schwarzpigmenten sowie auch herkömmlichen Glanzpigmenten auf der Basis von metalloxidbeschichteten Glimmer- und Metallpigmenten und plättchenförmigen Eisenoxiden verwenden.

Die erfindungsgemäßen metallischen Glanzpigmente sind kostengünstig herzustellen. Sie weisen ein außergewöhnlich hohes Deckvermögen auf und bieten somit vielfältige Vorteile für deren Verwendung, beispielsweise als Lack in der Automobil- und Fahrzeugindustrie. Die metallischen Glanzpigmente gemäß der vorliegenden Erfindung weisen darüber hinaus eine geringe Entflammbarkeit auf. Daher erfüllen sie selbst strenge Brandschutzvorschriften und Sicherheitsvorgaben.
Figuren 1 und 2 zeigen Ergebnisse von Verbrennungstests verschiedener beschichteter Aluminiumplättchen mit unterschiedlichem Gewichtsanteil an Aluminium (Figur 1) und Eisen(III)oxid (Figur 2) (nicht erfindungsgemäß). Die Ermittlung des anwendungstechnischen Wertes für das Brennverhalten (y-Achse) in Abhängigkeit vom Anteil an Aluminium (Figur 1) beziehungsweise Eisen(III)oxid (Figur 2) in den beschichteten Substratplättchen erfolgt durch Bewertung des Verhaltens der Probe in dem nachstehend beschriebenen Verbrennungstest.
Figur 3 zeigt ein beschichtetes Aluminiumplättchen (nicht erfindungsgemäß). Das Aluminiumplättchen weist eine sehr gleichmäßige Dicke auf und ist von einer SiO₂-Schicht (Beschichtung A, hell) sowie einer Eisenoxidschicht (Beschichtung B, dunkel) umhüllt.

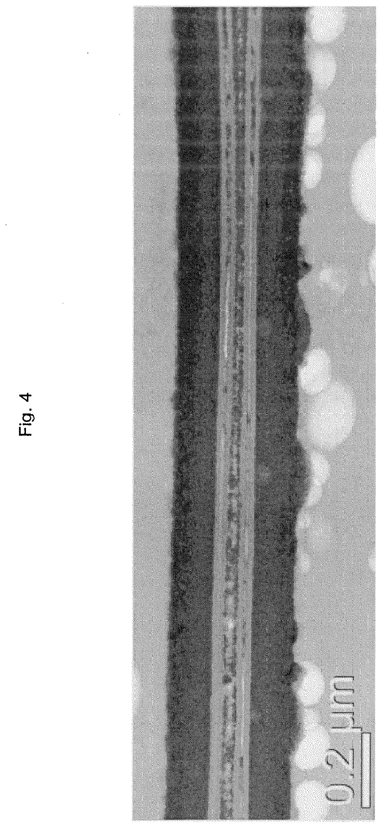

Figur 4 zeigt ein beschichtetes Aluminiumplättchen mit Aluminiumkern, SiO₂-Schicht (Beschichtung A, hell) und Eisenoxidschicht (Beschichtung B, dunkel) (nicht erfindungsgemäß).

Die nachstehenden Beispiele dienen der weiteren Erläuterung.

### Beispiel 1 (dünne Aluminiumplättchen mit dicker Eisenoxidbeschichtung) (nicht erfindungsgemäß)

Zunächst wurden 50 g Al-Plättchen (Dicke zwischen 20 nm und 30 nm, d50 = 12 µm) mittels Sol-Gel-Verfahren unter Verwendung von Tetraethylorthosilikat (TEOS) mit 10 g SiO₂ beschichtet. In einem Rundkolben mit Rückflußkühler und Rührer wurden diese Al-Plättchen mit 500 mL deionisiertem Wasser versetzt und unter Rühren auf 75°C erwärmt. Der pH-Wert wurde durch Hinzufügen einer 10%igen NaOH-Lösung auf einen Wert von 3,2 eingestellt. Zu dem Reaktionsgemisch wurden 1016 g einer 20%igen FeCl₃-Lösung gegeben, wobei der pH-Wert durch gleichzeitige Zugabe einer 10%igen NaOH-Lösung im Wesentlichen konstant bei 3,2 gehalten wurde. Nach vollständiger Zugabe der FeCl₃-Lösung wurde das Gemisch für weitere 15 Minuten gerührt, um eine vollständige Fällung zu gewährleisten. Der pH-Wert wurde dann durch Zutropfen einer 10%igen NaOH-Lösung über eine Dauer von 30 Minuten auf einen Wert von 7,0 erhöht. Nach 30 Minuten weiteren Rührens wurde das beschichtete Pigment von der überstehenden Reaktionslösung durch Filtrieren abgetrennt und gewaschen, bis es salzfrei war. Die erhaltenen beschichteten Aluminiumplättchen wurden 215 Minuten bei 250°C getrocknet und mit einem Sieb (Maschenweite 25 µm) gesiebt. Das erhaltene Produkt wurde einer Bewertung seiner Farbeigenschaften und einem Brandtest, wie nachstehend beschrieben, unterzogen.

### Beispiel 2 (dünne Aluminiumplättchen mit dünner Eisenoxidbeschichtung) (nicht erfindungsgemäß)

In diesem Beispiel wurden analog dem Verfahren von Beispiel 1 beschichtete Aluminiumplättchen hergestellt, mit dem Unterschied, dass statt 1016 g lediglich 102 g der 20%igen FeCl₃-Lösung eingesetzt wurden. Das erhaltene Produkt wurde einer Bewertung seiner Farbeigenschaften und einem Brandtest, wie unten beschrieben, unterzogen.

### Beispiel 3 (dünne Aluminiumplättchen mit dicker Eisenoxidbeschichtung und Titanoxidbeschichtung) (nicht erfindungsgemäß)

Dieses Beispiel wurde bis einschließlich dem fünfzehnminütigen Rühren nach beendeter Zugabe der FeCl₃-Lösung analog zu Beispiel 1 durchgeführt. Darauf wurde der pH-Wert durch Zugabe 10%iger HCl-Lösung auf 2,0 eingestellt. Dem Reaktionsgemisch wurden 412 g einer 30%igen TiCl₄-Lösung zugegeben, wobei der pH-Wert durch gleichzeitiges Hinzufügen einer 10%igen NaOH-Lösung im Wesentlichen konstant bei 2,0 gehalten wurde. Nach vollständiger Zugabe der TiCl₄-Lösung wurde das Gemisch für weitere 15 Minuten gerührt, um eine vollständige Fällung zu gewährleisten. Der pH-Wert wurde dann durch Zutropfen einer 10%igen NaOH-Lösung über eine Dauer von 30 Minuten auf einen Wert von 7,0 erhöht. Nach 30 Minuten weiteren Rührens wurde das beschichtete Pigment von der überstehenden Reaktionslösung durch Filtrieren abgetrennt und gewaschen, bis es salzfrei war. Die erhaltenen beschichteten Aluminiumplättchen wurden bei 250°C getrocknet und mit einem Sieb (Maschenweite 25 µm) gesiebt. Das erhaltene Produkt wurde einer Bewertung seiner Farbeigenschaften und einem Brandtest, wie nachstehend beschrieben, unterzogen.

### Vergleichsbeispiel (dicke Aluminiumplättchen mit Eisenoxidbeschichtung)

Zunächst wurden 50 g Al-Plättchen (Dicke zwischen 150 nm und 300 nm, d50 = 18 µm) mittels Sol-Gel-Verfahren unter Verwendung von Tetraethylorthosilikat (TEOS) mit 8,8 g SiO₂ beschichtet. In einen Rundkolben mit Rückflußkühler und Rührer wurden diese Al-Plättchen mit 500 mL deionisiertem Wasser versetzt und unter Rühren auf 75°C erwärmt. Der pH-Wert wird durch Hinzufügen einer 10%igen NaOH-Lösung auf einen Wert von 3,2 eingestellt. Zu dem Reaktionsgemisch wurden 660 g einer 20%igen FeCl₃-Lösung gegeben, wobei der pH-Wert durch gleichzeitige Zugabe einer 10%igen NaOH-Lösung im Wesentlichen konstant bei 3,2 gehalten wurde. Nach vollständiger Zugabe der FeCl₃-Lösung wurde das Gemisch für weitere 15 Minuten gerührt, um eine vollständige Fällung zu gewährleisten. Der pH-Wert wurde dann durch Zutropfen einer 10%igen NaOH-Lösung über eine Dauer von 30 Minuten auf einen Wert von 7,0 erhöht. Nach 30 Minuten weiteren Rührens wurde das beschichtete Pigment von der überstehenden Reaktionslösung durch Filtrieren abgetrennt und gewaschen bis es salzfrei war. Die erhaltenen beschichteten Aluminiumplättchen wurden bei 250°C getrocknet und mit einem Sieb (Maschenweite 40 µm) gesiebt. Das erhaltene Produkt wurde einer Bewertung seiner Farbeigenschaften und einem Brandtest, wie unten beschrieben, unterzogen.

### Verbrennungstest

Je 20 g der hergestellten Pigmente wurden mit 13,3 g Testbenzin gründlich durchmischt. Auf eine Glasplatte wurden 2 g dieses Gemisches gegeben und entflammt. Dieser Verbrennungstest wird als Video aufgezeichnet. Das Brandverhalten wird nach einer Skala von 0 bis 5 bewertet, wobei 0 bedeutet, dass lediglich das Testbenzin bei ruhigem Brandverhalten abbrennt, ohne dass eine weitere Reaktion eintritt.
1: vereinzelte Funken während des Lösemittelbrands
2: leichte Funkenentwicklung während des Lösemittelbrands
3: mittelstarke Funkenentwicklung während des Lösemittelbrands
4: starke Funkenentwicklung und kleine Explosionen bzw. Verpuffungen während des Lösemittelbrands, Durchglühen der Probe nach dem Abbrennen des Lösemittels
5: starke Funkenentwicklung und Explosionen bzw. Verpuffungen während des Lösemittelbrands, sowie vollständige Umsetzung der Probe nach dem Abbrennen des Lösemittels

Zur Messung des Gesamtfarbabstandes ΔE wurde auf jeweils eine schwarze und eine weiße Oberfläche eine Lackschicht, die das zu untersuchende erfindungsgemäße metallische Glanzpigment mit einem Massenanteil von 18 Gew.-% (Trockengewicht) enthielt, aufgetragen. Die Schichtdicke der getrockneten Lackierung betrug 15 µm. Danach wurde der Gesamtfarbabstand ΔE zwischen den Lackierungen auf weißem und schwarzem Grund bestimmt. Die Ergebnisse der Messungen sind in Tabelle 1 gezeigt.

Tabelle 1 zeigt Resultate für verschiedene metallische Glanzpigmente. Die unbeschichteten Substratplättchen bestehen aus Aluminiummetall. Tests Nummer 1 bis 12 wurden entsprechend den Vorschriften aus den Beispielen 1, 2 bzw. 3 mit den nötigen Modifikationen zur Einstellung der individuell spezifischen Parameter (z.B. Dicke der Beschichtungen A, B und, falls vorhanden, C) hergestellt. Tests Nummer 1 bis 12 sind Vergleichsbeispiele. Zusätzlich zu den Tests Nummer 1 bis 12 sind in Tabelle 1 Werte für die käuflich erhältlichen Produkte Paliochrom L2800 (Firma BASF) und Meoxal Orange (Firma Merck) als Vergleichsbeispiele dargestellt.

Aus Tabelle 1 und Figur 1 ist ersichtlich, dass durch einen Gehalt an Aluminiummetall der beschichteten Substratplättchen von insbesondere gleich oder weniger als 20 Gew.-% ein Pigment, das nicht brennbar oder explosionsgefährlich ist, bereitgestellt werden kann. Dies entspricht im Verbrennungstest einem Ergebnis von 1 oder 0.

Aus Tabelle 1 und Figur 2 ist ersichtlich, dass durch einen Fe₂O₃-Gehalt der beschichteten Substratplättchen von insbesondere gleich oder größer 65 Gew.-% ein Pigment, das nicht brennbar oder explosionsgefährlich ist, bereitgestellt werden kann. Dies entspricht im Verbrennungstest einem Ergebnis von 1 oder 0.

Darüber hinaus zeigt Tabelle 1, dass die metallischen Glanzpigmente einen besonders geringen Farbabstand ΔE und damit ein besonders hohes Deckvermögen aufweisen. Die in der Tabelle angegebenen Anteile beziehen sich auf Gew.-%.

**Tabelle 1**

| **Nr** | **Al Anteil** | **SiO₂ Anteil** | **Fe₂O₃ Anteil** | **TiO₂ Anteil** | **Anwendungstechnischer Wert für Brennverhalten** | **ΔE** | **Verwendung von erfindungsgemäßem Substrat** |
|---|---|---|---|---|---|---|---|
| 1 | 37 | 14 | 39 | 0 | 5 | 0,8 | ja |
| 2 | 34 | 21 | 35 | 0 | 5 | 0,6 | ja |
| 3 | 26 | 30 | 30 | 0 | 5 | 0,8 | ja |
| 4 | 22 | 25 | 45 | 0 | 4 | 0,2 | ja |
| 5 | 11 | 13 | 68 | 0 | 1 | 0,7 | ja |
| 6 | 6 | 8 | 73 | 0 | 0 | 0,4 | ja |
| 7 | 4 | 5 | 72 | 0 | 0 | 3,3 | ja |
| 8 | 4 | 6 | 45 | 30 | 0 | 15,4 | ja |
| 9 | 37 | 7 | 49 | 0 | 5 | 0,4 | ja |
| 10 | 51 | 6 | 32 | 0 | 4 | 5,5 | nein |
| 11 | 64 | 4 | 20 | 0 | 2 | 11,2 | nein |
| 12 | 8 | 3 | 76 | 0 | 0 | 0,8 | ja |
| Paliocrom L2800 | 69 | 1 | 26,6 | nb | 3 | 12,0 | nein |
| Meoxal Orange | 27 | 11 | 50,9 | nb | 5 | 25,0 | nein |

## Patentansprüche

1. Metallische Glanzpigmente auf der Basis von beschichteten Aluminium-Substratplättchen,
wobei die Aluminium-Substratplättchen eine Dicke von 5 bis 30 nm aufweisen, monolithisch aufgebaut und gegebenenfalls passiviert sind und von einer Beschichtung B umhüllt sind,
wobei die Beschichtung B aus Fe₂O₃ in einer Dicke von 50 bis 300 nm aufgebaut ist, und
wobei zwischen der Oberfläche der Aluminium-Substratplättchen und der Beschichtung B eine weitere, die Substratplättchen umhüllende Beschichtung A aus SiO₂ in einer Dicke von 5 bis 50 nm vorhanden ist,
wobei die Substratplättchen eine weitere Beschichtung C, die von der darunterliegenden Beschichtung B verschieden ist, aus mindestens einem Metalloxid(hydrat), ausgewählt aus Silicium(di)oxid, Siliciumoxidhydrat, Aluminiumoxid, Aluminiumoxidhydrat, Zinkoxid, Zinnoxid, Titandioxid, Zirkonoxid oder Chrom(III)oxid, aufweisen, und
wobei als Aluminium-Substratplättchen Al-VMPs verwendet werden.

2. Verfahren zur Herstellung der metallischen Glanzpigmente nach Anspruch 1, umfassend die Schritte
des Bereitstellens von gegebenenfalls passivierten Aluminium-Substratplättchen,
des Beschichtens der Aluminium-Substratplättchen durch hydrolytische Zersetzung einer oder mehrerer organischer Metallverbindungen und/oder durch Fällung eines oder mehrerer gelöster Metallsalze.

3. Verwendung der metallischen Glanzpigmente nach Anspruch 1 zur Einfärbung von Lacken, Druckfarben, Tinten, Kunststoffen, Gläsern, keramischen Produkten und Zubereitungen der dekorativen Kosmetik.

## Claims

1. Metallic luster pigments based on coated aluminum substrate platelets,
wherein the aluminum substrate platelets have a thickness of 5 to 30 nm, are of monolithic structure and have optionally been passivated and are encased by one coating B,
wherein the coating B is composed of Fe₂O₃ in a thickness of 50 to 300 nm, and wherein between the surface of the aluminum substrate platelets and the coating B there is one further coating A which encases the substrate platelets and is composed of SiO₂ in a thickness of 5 to 50 nm,
wherein the substrate platelets have a further coating C which is different from the underlying coating B, of at least one metal oxide (hydrate) selected from silicon (di)oxide, silicon oxide hydrate, aluminum oxide, aluminum oxide hydrate, zinc oxide, tin oxide, titanium dioxide, zirconium oxide or chromium(III) oxide, and wherein AI-VMP are used as aluminum substrate platelets.

2. A process for producing metallic luster pigments according to claim 1, comprising the steps of:
providing optionally passivated aluminum substrate platelets,
coating the aluminum substrate platelets by hydrolytic decomposition of one or more organic metal compounds and/or by precipitation of one or more dissolved metal salts.

3. Use of the metallic luster pigments according to claim 1 for coloring paints, printing inks, other inks, plastics, glasses, ceramic products and formulations for decorative cosmetics.

## Revendications

1. Pigments à lustre métallique à base de plaquettes de substrat en aluminium revêtues,
dans lesquels les plaquettes de substrat en aluminium ont une épaisseur de 5 à 30 nm, sont de structure monolithique et ont éventuellement été passivées et sont recouvertes d'un revêtement B,
dans lesquels le revêtement B est composé de Fe₂O₃ selon une épaisseur de 50 à 300 nm, et
dans lesquels entre la surface des plaquettes de substrat en aluminium et le revêtement B se trouve un autre revêtement A qui enveloppe les plaquettes de substrat et est composé de SiO₂ selon une épaisseur de 5 à 50 nm,
dans lesquels les plaquettes de substrat ont un revêtement supplémentaire C qui est différent du revêtement sous-jacent B, constitué d'au moins un oxyde de métal (hydraté) sélectionné parmi le (di)oxyde de silice, l'oxyde de silice hydraté, l'oxyde d'aluminium, l'oxyde d'aluminium hydraté, l'oxyde de zinc, l'oxyde d'étain, le dioxyde de titane, l'oxyde de zircone ou l'oxyde de chrome (III), et
dans lesquels AI-VMP sont utilisés comme plaquettes de substrat d'aluminium.

2. Procédé de production de pigments à lustre métallique selon la revendication 1, comprenant les étapes de :
fournir des plaquettes de substrat en aluminium éventuellement passivées,
revêtir les plaquettes du substrat d'aluminium par décomposition hydrolytique d'un ou plusieurs composés métalliques organiques et/ou par précipitation d'un ou plusieurs sels métalliques dissous.

3. Utilisation des pigments à lustre métallique selon la revendication 1 pour colorer des peintures, des encres d'imprimerie, d'autres encres, des matières plastiques, des verres, des produits céramiques et des formulations pour cosmétiques décoratifs.
